# EUROPÄISCHE PATENTSCHRIFT

(11) **EP 3 507 355 B1**
(45) Veröffentlichungstag und Bekanntmachung des Hinweises auf die Patenterteilung: **21.07.2021**
(21) Anmeldenummer: 17762082.0
(22) Anmeldetag: 30.08.2017
(51) Int. Cl.: C12M 1/00, C12M 1/12

(54) **PHOTOBIOREAKTOR UND VERFAHREN ZUR KULTIVIERUNG VON MIKROALGEN**
PHOTOBIOREACTOR AND METHOD FOR CULTIVATING MICROALGAE
PHOTOBIOREACTEUR ET PROCEDE DE CULTURE DE MICRO-ALGUES

(30) Priorität: 30.08.2016 EP 16186440
(43) Veröffentlichungstag der Anmeldung: 10.07.2019
(73) Patentinhaber: Beco Invest B.V., 3992 LX Houten (NL)
(72) Erfinder: EMMINGER, Franz, 2410 Hainburg (AT); FLUCH, Silvia, 7121 Weiden/See (AT)
(74) Vertreter: Redl, Gerda
(86) Internationale Anmeldenummer: PCT/EP2017/071707
(87) Internationale Veröffentlichungsnummer: WO 2018/041863

(56) Entgegenhaltungen:
- WO-A1-2015/179888
- WO-A2-2009/051478
- US-A1- 2011 027 875

## Beschreibung

Das Gebiet der vorliegenden Erfindung ist das der Photobioreaktoren zur Kultivierung phototropher Mikroorganismen wie beispielsweise Mikroalgen.

Phototrophe Mikroorganismen wie Mikroalgen werden vor allem ihrer wertvollen Inhaltsstoffe wegen kultiviert, die z.B. in der Herstellung von medizinischen Präparaten, Nahrungs- und Futtermitteln, Nahrungsergänzungsmitteln, und Kosmetika benötigt werden. Zu diesen Inhaltsstoffen gehören unter anderem ungesättigte Fettsäuren (z.B. Omega-3 und Omega-6-Fettsäuren), Antioxidantien wie Astaxanthin und Lutein, und Chlorophyll. Die Zusammensetzung der Inhaltsstoffe ist von der kultivierten Art von phototrophen Mikroorganismen abhängig. Vorwiegend wird eine Reinkultur von phototrophen Mikroorganismen herangezogen, um eine gewisse Qualität bzw. Mindestkonzentration der gewünschte Inhaltsstoffe gewährleisten zu können.

Üblicherweise werden die Photosynthese betreibenden Mikroorganismen unter Einwirkung von Licht (z.B. Sonnenlicht) im nährstoffhaltigen Kulturmedium in einem Photobioreaktor kultiviert, und anschließend aufkonzentriert und verarbeitet. Aus der erhaltenen Biomasse können die Inhaltsstoffe zur Weiterverarbeitung gewonnen werden bzw. kann die Biomasse selbst z.B. als Futtermittel oder Dünger verwendet werden.

Eine im Stand der Technik bekannte Bauweise von Photobioreaktoren zur Kultivierung phototropher Mikroorganismen ist die des "vertikalen Rohr-Reaktors" ("vertical tubular reactor"), bei dem aufrechte, parallele, im Wesentlichen transparente Rohre (mitunter auch als Säulen bezeichnet) in Serie geschaltet sind und zumeist mäanderförmig vom Kulturmedium mit den phototrophen Mikroorganismen durchflossen werden. Ein Photobioreaktor dieser Bauweise ist beispielsweise aus der US 2014/0242681 A1 bekannt.

Im Allgemeinen sind die Rohre eines Photobioreaktors insbesondere im Dauerbetrieb anfällig dafür, mit der Zeit zu verschmutzen. Dabei lagern sich oft Rückstände aus der Kultivierung (beispielsweise aus Überresten toter Mikroorganismen) an der Innenseite der Rohre an. Dies kann zum einen die Lichtausbeute mindern (da diese Rückstände typischerweise Licht absorbieren) und zum anderen eine ständige Gefahr in Bezug auf Kontamination des Kulturmediums mit unerwünschten Mikroorganismen darstellen, die in den Rückständen einen geeigneten Nährboden finden. Durch verminderte Lichtausbeute bzw. Kontamination kann sich wiederum die Ausbeute aus der Kultivierung deutlich verringern. Des weiteren kann es insbesondere für sensible Bereiche wie die Herstellung von medizinischen Präparaten oder Nahrungsergänzungsmitteln von hoher Bedeutung für die Produktsicherheit sein, Kontaminationen der Kultur mit unerwünschten (und mitunter sogar toxischen) Mikroorganismen möglichst auszuschließen.

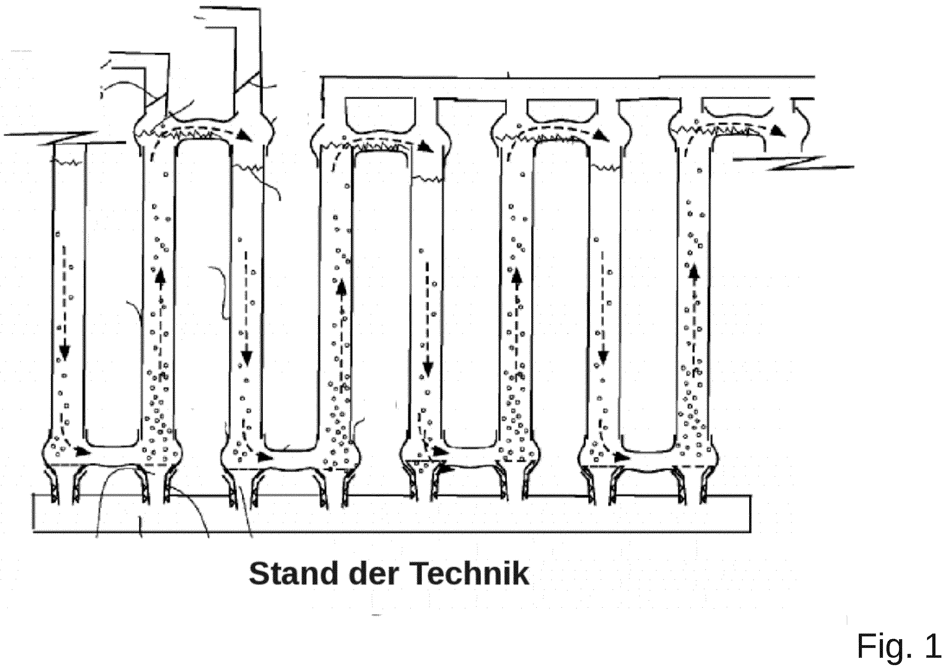

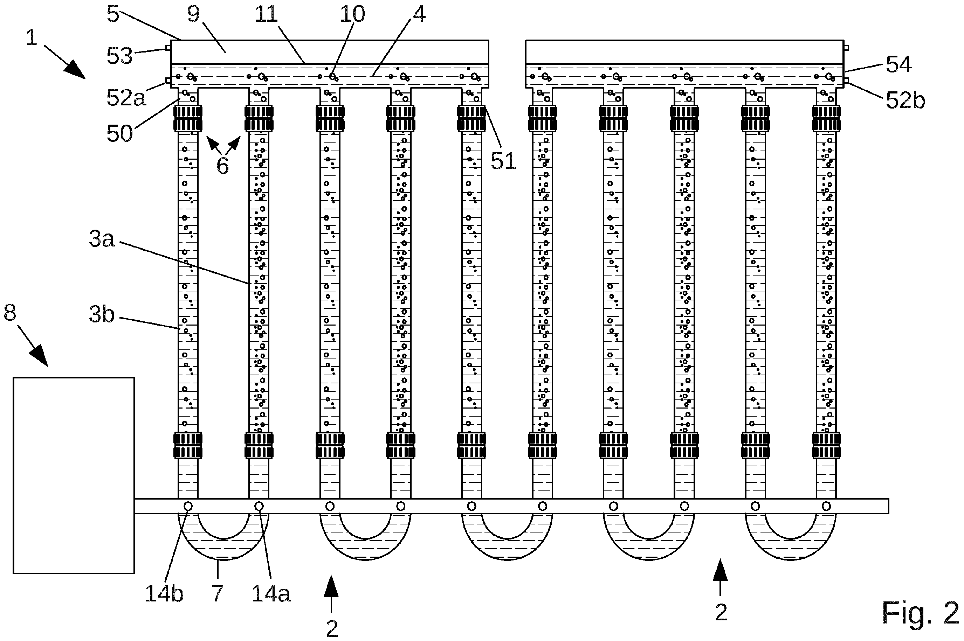

Die WO 2009/051478 A2 betrifft einen Photobioreaktor mit Reinigungssystem. Zwischen Stationen kann ein Reinigungswerkzeug (z.B. ein zylindrischer Schwamm, vgl. auch Fig. 2-5 des Dokuments) zur Reinigung der Reaktor-Rohrinnenwände hin und her gepumpt werden (vgl. S. 12, 2. Absatz). Im Dokument sind die Rohre des Photobioreaktors als über U-förmige Verbindungsstücke verbunden (vgl. Fig. 1, Bezugszeichen 5, des Dokuments) sowie nicht als begast offenbart.

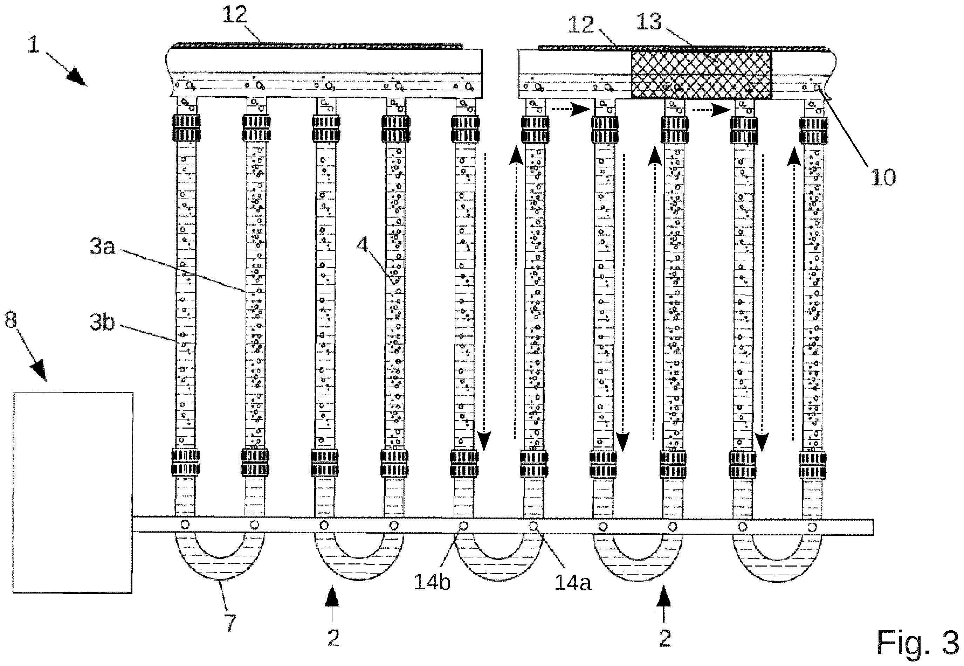

Die WO 2015/179888 A1 offenbart einen Reaktor des vertikalen Typs. Dieser Reaktor besteht aus mindestens einem Reaktorelement, das aus mindestens zwei aufrechten, verbundenen Röhren oder Kammern gebildet ist, wobei die einzelnen Reaktorelemente an der Unterseite an jeweils den äußersten Röhren oder Kammern Ein- und Auslassöffnungen aufweisen, wobei der Einlassöffnung jeweils eine gegen die Richtung der Schwerkraft, also ein aufsteigender Ast, und der Auslassöffnung eine in Richtung der Schwerkraft, also ein abfallender Ast, zugeordnet ist, dass alle Anschlüsse im unteren Bereich, insbesondere die Einlassöffnung, die Auslassöffnung und der Einbringungseinlass, mit dem Reaktionsmedium umspült sind. Aus Fig. 1 bzw. Fig. 3 des Dokuments lässt sich entnehmen, dass die Rohre oder Kammern im Reaktorelement "in Serie geschaltet sind" (vgl. auch S. 14, Z 19, des Dokuments), d.h. dass nie mehr als zwei Rohre bzw. Kammern im Reaktorelement als direkt miteinander miteinander verbunden offenbart sind. Gemäß einer Ausgestaltung ist das Reaktorelement an der Oberseite mit einem Abschlusselement verschlossen, wobei das Abschlusselement abnehmbar ausgebildet ist, um die Anlage reinigen zu können (S. 16, 1. Absatz, des Dokuments).

Einen weiteren Lösungsansatz für das oben geschilderte Problem vermeint der Stand der Technik im "Einweg"-Photobioreaktor gefunden zu haben. Bei diesem sind die Reaktorbestandteile wie z.B. Rohre aus vergleichsweise kostengünstigen Kunststoffmaterial gefertigt, das bereits nach kurzem Betrieb wieder ausgetauscht wird.

So bezieht sich die US 2011/0027875 A1 auf einen kostengünstigen, vertikalen Photobioreaktor für den Produktionseinsatz. Gemäß den dargestellten Ausführungsformen des Dokuments sind bei diesem Photobioreaktor im wesentlichen vertikal orientierte, parallele, transparente Säulen oder Rohre durch untere und obere U-förmige Verbindungsstücke miteinander verbunden, so dass der Photobioreaktor im Betrieb in einem geschlossenen Kreislauf von Kulturmedium mit Mikroalgen mäanderförmig durchflossen werden kann. Die Rohre bzw. Säulen (bzw. das Kulturmedium darin) werden mit Kohlendioxid begast, welches bekanntlich von den Mikroalgen zu Sauerstoff umgesetzt wird. Die unteren und oberen U-förmigen Verbindungsstücke können jeweils mit einem Gasverteilrohr ("gas manifold") verbunden sein (vgl. Fig. 1, die den unteren Ausschnitt von Fig. 1 des genannten Dokuments wiedergibt). Als Material für die Säulen oder Rohre wird ein flexibles Polyethylen mit niedriger Dichte ("flexible LDP bag material") vorgeschlagen. Gemäß der Absätze [0087]-[0090] wird bei ungünstigem Zustand der Mikroalgenkultur dieses Material verworfen, die U-förmigen Verbindungsstücke werden vom Gasverteilrohr getrennt und z.B. in einem Geschirrspüler gereinigt.

Diese Herangehensweise an die Reinigung bzw. das Sauberhalten von Photobioreaktoren im Stand der Technik ist mit vielen Nachteilen verbunden, unter anderem verursacht sie (vor allem bei großen Produktionsanlagen) hohen manuellen Arbeitsaufwand und lange Stehzeiten, welche die Ausbeute des Photobioreaktors mit der Zeit schmälern. Des Weiteren wirkt sich der hohe Verbrauch an Material (üblicherweise Kunststoff) nachteilig auf die ökologische Gesamtbilanz des Photobioreaktorbetriebs aus.

Es ist Aufgabe der vorliegenden Erfindung, die Reinigung bzw. das Sauberhalten eines Photobioreaktors zur Kultivierung phototropher Mikroorganismen zu vereinfachen. Dadurch können die Lebensdauer bzw. Ausbeute des Photobioreaktors erhöht und die Betriebskosten verringert werden.

Die vorliegende Erfindung stellt einen Photobioreaktor zur Kultivierung phototropher Mikroorganismen, mit einem Reaktorelement, das eine Vielzahl von Steig- und Fallrohren für flüssiges Kulturmedium, welches die Mikroorganismen beinhaltet, und ein Verteilrohr aufweist, zur Verfügung. Die Steig- und Fallrohre sind jeweils an ihrem oberen Ende mit dem Verteilrohr flüssigkeitsdurchlässig verbunden. Zumindest eines der Steig- und eines der Fallrohre sind zusätzlich miteinander durch ein Verbindungsstück flüssigkeitsdurchlässig verbunden (bzw. angeschlossen). Der Photobioreaktor weist ferner eine Einrichtung zum Einleiten von Gas in zumindest ein Steigrohr auf. Erfindungsgemäß ist der Photobioreaktor derart ausgelegt, dass im Betriebszustand im Verteilrohr sowohl Kulturmedium als auch, oberhalb des Kulturmediums, ein Gasraum zur Aufnahme von aus dem Kulturmedium aufsteigenden Gasblasen vorhanden sind, wobei im Verteilrohr eine Grenzfläche zwischen dem Kulturmedium und dem Gasraum angeordnet ist. Dadurch wird die genannte Aufgabe gelöst.

Dementsprechend stellt die Erfindung auch ein Verfahren zur Kultivierung phototropher Mikroorganismen zur Verfügung. Dieses umfasst das Belichten eines Photobioreaktors, wobei der Photobioreaktor versehen ist mit einem Reaktorelement, das eine Vielzahl von Steig- und Fallrohren für flüssiges Kulturmedium, welches die Mikroorganismen beinhaltet, und ein Verteilrohr aufweist, wobei die Steig- und Fallrohre jeweils an ihrem oberen Ende mit dem Verteilrohr flüssigkeitsdurchlässig verbunden sind, wobei zumindest eines der Steig- und eines der Fallrohre zusätzlich miteinander durch ein Verbindungsstück flüssigkeitsdurchlässig verbunden sind, und wobei der Photobioreaktor in den Steig- und Fallrohren flüssiges Kulturmedium mit den Mikroorganismen beinhaltet, welches zumindest teilweise begast wird. Im Verteilrohr sind sowohl flüssiges Kulturmedium mit den Mikroorganismen als auch, oberhalb des Kulturmediums, ein Gasraum vorhanden, wobei im Verteilrohr eine Grenzfläche zwischen dem Kulturmedium und dem Gasraum ausgebildet wird, und der Gasraum aus dem Kulturmedium aufsteigende Gasblasen aufnimmt. Auch dadurch wird die genannte Aufgabe gelöst.

Es hat sich während der Entwicklung der vorliegenden Erfindung herausgestellt, dass gerade die mit der Grenzfläche zwischen Kulturmedium und Gasraum in Berührung befindlichen Innenoberflächen eines Photobioreaktors (bzw. der "Grenzflächenraum" in der näheren Umgebung der Grenzfläche) anfällig für Verschmutzungen sind, wobei sich mit der Dauer des Betriebes üblicherweise immer mehr Verschmutzungen anlagern. Im Bereich der Grenzfläche (die oft über die Betriebsdauer geringfügig in der Höhe variiert) kann es immer wieder zu Schaumbildung und Antrocknungen von Rückständen der phototrophen Mikroorganismen kommen. Insbesondere wenn eine Begasung des Photobioreaktors vorgesehen ist, lässt sich das Vorkommen solcher verschmutzungsanfälligen Grenzflächen (bzw. Grenzflächenräume) im Photobioreaktor kaum vermeiden. Zusätzlich verursachen die aus dem Kulturmedium aufsteigenden Gasblasen, dass die Innenoberfläche des Photobioreaktors mit Kulturmedium bespritzt wird und diese Spritzer über die Zeit ebenfalls antrocknen und dadurch Verschmutzungen bilden. Wenn sich die genannten Grenzflächen, wie in Fig. 1 gezeigt (siehe insbesondere die obersten gebogenen Pfeile in Fig. 1), an schwieriger zugänglichen Bereichen befindet, bleibt zur Reinigung oft nichts als die vorübergehende Demontage des Photobioreaktors, wie z.B. in der US 2011/0027875 A1 offenbart.

Die vorliegende Erfindung beruht nun unter anderem auf der Erkenntnis, dass die Reinigung bzw. das Sauberhalten des Photobioreaktors wesentlich erleichtert wird, indem die genannten Grenzflächen von schwieriger zugänglichen Bereichen durch die erfindungsgemäße Bauweise bzw. den erfindungsgemäßen Betrieb des Photobioreaktors sozusagen in ein (leichter zugängliches) Verteilrohr verschoben und dort zu einer Grenzfläche zusammengefasst werden.

Es hat sich unerwarteterweise herausgestellt, dass im erfindungsgemäßen Photobioreaktor dennoch eine mäanderförmige Strömung aufrecht erhalten werden kann, obwohl im Stand der Technik zur Gewährleistung der mäanderförmigen Strömung üblicherweise U-förmige Verbindungsstücke sowohl am oberen als auch am unteren Ende der Steig- bzw. Fallrohre vorgesehen werden (vgl. z.B. die US 2014/0242681 A1 oder die US 2011/0027875 A1). Daher werden in einer bevorzugten Ausführungsform des erfindungsgemäßen Verfahrens die Steig- und Fallrohre mäanderförmig vom Kulturmedium durchströmt. Ein Vorteil der mäanderförmigen Strömung ist, dass die Reifung der phototrophen Mikroorganismen geregelter abläuft, weil Mikroorganismen ähnlichen Reifegrades im Kulturmedium in geringeren Ausmaß voneinander abgeschieden als dies bei chaotischerer Strömung der Fall wäre (z.B. wenn Turbulenzen oder verschiedene Strömungspfade vorhanden sind). So können Mikroorganismen ähnlichen Reifegrades auch einfacher gemeinsam aus dem Photobioreaktor entnommen werden.

Als besonders vorteilhaft für die Stabilisierung der mäanderförmigen Strömung hat sich erwiesen, wenn im Photobioreaktor zumindest zwei Reaktorelemente vorgesehen sind, wobei ein Fallrohr des ersten Reaktorelements mit einem Steigrohr des zweiten Reaktorelements miteinander durch ein Verbindungsstück flüssigkeitsdurchlässig verbunden sind.

In einer bevorzugten Ausführung wird im Betriebszustand eine mäanderförmige Strömung des Kulturmediums durch die Steig- und Fallrohre dadurch erreicht, dass das Kulturmedium im mit dem Fallrohr durch das Verbindungsstück verbundenen Steigrohr stärker begast wird als in dem Fallrohr. Es kann in diesem Zusammenhang auch von einem "Gaslift"-Effekt gesprochen werden. Ein Vorteil dieser Ausführung ist, dass im Betrieb auf Pumpen (die für die phototrophen Mikroorganismen durch die von ihnen verursachten Scherkräfte üblicherweise schädlich sind) zumindest weitgehend verzichtet werden kann. Ein weiterer Vorteil besteht darin, dass die inneren Oberflächen der Steig- bzw. Fallrohre durch den häufigen Kontakt mit aufsteigenden Gasblasen sauberer gehalten werden.

Zweckmäßigerweise wird also im Betriebszustand des Photobioreaktors eine Agitation und/oder ein Fluss des Kulturmediums im Wesentlichen durch die im Kulturmedium aufsteigenden Gasblasen verursacht.

In einer besonders bevorzugten Ausführungsform ist am Verteilrohr eine Einrichtung zur Führung einer im Verteilrohr beweglichen Instandhaltungsvorrichtung vorgesehen. Dabei kann es sich beispielsweise um eine Führungsschiene, einen Führungsdraht, oder eine Leiste mit Positionierungsmarkierungen, die von der Instandhaltungsvorrichtung abgelesen werden können, handeln. Insbesondere wenn die Einrichtung zur Führung magnetisch ist, kann sie auch auf der Außenseite des Verteilrohres angebracht sein. Die Instandhaltungsvorrichtung kann die innere Oberfläche des Verteilrohres, insbesondere im Bereich der Grenzfläche, mit Hilfe von Sprühdüsen, Bürsten und/oder Wischblättern von gegebenenfalls eingetrockneten Rückständen befreien, d.h. reinigen bzw. sauberhalten.

In einer bevorzugten Ausführung des erfindungsgemäßen Verfahrens wird die innere Oberfläche des Verteilrohres von der beweglichen Instandhaltungsvorrichtung, vorzugsweise in voreinstellbaren Zeitabständen, gereinigt. Vorteilhafterweise geschieht dies während des Betriebes, so dass Einbußen bei der Ausbeute möglichst vermieden werden. Dabei ist es zweckmäßig, wenn die Instandhaltungsvorrichtung innerhalb des Verteilrohres umströmbar ist.

Durch die erfindungsgemäße Bauweise wird ermöglicht, dass statt Einweg-Kunststoffkomponenten (hochwertigere) Glasbauteile verwendet werden können, unter anderem auch weil das Risiko für Glasbruch sinkt, wenn eine Demontage zur Reinigung weniger oft erforderlich ist. Somit bestehen in einer bevorzugten Ausführungsform des Photobioreaktors die Steig- und Fallrohre und/oder das Verteilrohr im Wesentlichen aus Glas. Dadurch wird unter anderem die Langlebigkeit bzw. Widerstandsfähigkeit des Photobioreaktors erhöht, insbesondere wenn Mikroalgen kultiviert werden, die ein Kulturmedium mit extremem pH-Wert benötigen. Hinzu kommt, dass sich Glas typischerweise mit der Zeit weniger aufraut als der üblicherweise verwendete Kunststoff, so dass die Reinigung bzw. das Sauberhalten vereinfacht wird. Das Glas kann beschichtet sein, wie beispielsweise in der DE 10 2009 029 792 A1 oder der WO 2011/015653 A2 offenbart.

Gemäß einer weiteren bevorzugten Ausführungsform ist für das mit dem Fallrohr durch das Verbindungsstück verbundene Steigrohr ein Einlass zur Begasung mit der Einrichtung vorgesehen, und für das mit dem Steigrohr durch das Verbindungsstück verbundene Fallrohr ein weiterer Einlass zur Begasung mit der Einrichtung vorgesehen. Dadurch kann eine unterschiedlich starke Begasung der Steig- und Fallrohre ermöglicht werden. Bei Begasung über dieselbe Gasleitung kann dies z.B. durch Vorsehen eines Begasungseinlasses des Steigrohres mit einer oder mehreren Öffnungen (z.B. in Form von Membranen oder gesinterten Scheiben), die insgesamt durchlässiger sind als die des Begasungseinlasses des Fallrohres, erreicht werden. Alternativ kann das Steigrohr (d.h. der Begasungseinlass des Steigrohres) z.B. über eine erste Gasleitung mit einem Druck A begast werden, während das Fallrohr (d.h. der Begasungseinlass des Fallrohres) über eine zweite Gasleitung mit einem Druck B begast wird, wobei A größer als B ist.

Es ist besonders günstig, wenn zumindest einer der Einlässe zur Begasung im Verbindungsstück vorgesehen ist, weil dies im Aufbau des Photobioreaktors leichter zu bewerkstelligen ist. Das Verbindungsstück kann nämlich (durchgehend) aus leichter bearbeitbaren, intransparenten Materialien wie z.B. Edelstahl bestehen.

Der Photobioreaktor der vorliegenden Erfindung ist vorzugsweise vom Typ her ein Rohrreaktor ("tubular reactor"), genauer gesagt ein vertikaler Rohrreaktor ("vertical tubular reactor"), bzw. kein Plattenreaktor ("plate reactor" bzw. "flat panel reactor").

Erfindungsgemäß beträgt die genannte Vielzahl von Steig- bzw. Fallrohren des Reaktorelements, die mit dem selben Verteilrohr flüssigkeitsdurchlässig verbunden sind, zumindest drei (z.B. in der Anordnung Fallrohr - Steigrohr - Fallrohr oder Steigrohr - Fallrohr - Steigrohr entlang des Verteilrohres). Es ist im Sinne der Skalierbarkeit der Reinigung günstig, wenn die genannte Vielzahl zumindest vier, bevorzugt zumindest fünf, mehr bevorzugt zumindest zehn, noch mehr bevorzugt zumindest zwanzig, insbesondere zumindest dreißig oder gar zumindest vierzig beträgt. Vorteilhafterweise wird eine alternierende Anordnung von Steig- bzw. Fallrohren entlang des Verteilrohres gewählt (also z.B. Steigrohr - Fallrohr - Steigrohr - ... - Fallrohr - Steigrohr oder Steigrohr - Fallrohr - Steigrohr - ... - Fallrohr - Steigrohr - Fallrohr, oder Fallrohr - Steigrohr - Fallrohr - ... - Steigrohr). Zweckmäßigerweise ist die Positionierung von Ein- bzw. Auslässen (z.B. am oberen oder unteren Ende) im Photobioreaktor natürlich gemäß dieser Anordnung so zu wählen, dass ein Totvolumen vermieden wird. Für den Fachmann ist evident, dass durch Umdrehen des Flusses des Kulturmediums (z.B. durch Änderung der Begasung) ein Steigrohr zu einem Fallrohr werden kann bzw. umgekehrt. Vorteilhafterweise ergibt sich aus der Summe der Flussgeschwindigkeitsvektoren der im Verteilrohr herrschenden Strömungen des Kulturmediums (insbesondere wenn im Reaktorelement eine mäanderförmige Strömung geführt wird) ein Flussgeschwindigkeitsvektor, der im Wesentlichen parallel zur Längsachse des Verteilrohres ist (sozusagen eine Netto-Strömung entlang der Längsachse, vgl. auch die horizontalen gestrichelten Pfeile in Fig. 3).

Es ist günstig, wenn die Längsachsen der Steig- bzw. Fallrohre, gegebenenfalls unabhängig voneinander, in einem Winkel von mehr als 5°, bevorzugt von mehr als 20°, mehr bevorzugt von mehr als 40° oder gar von mehr als 60°, noch mehr bevorzugt von mehr als 70° oder gar von mehr als 80°, insbesondere von mehr als 85° oder gar von mehr als 87,5° zum Spiegel des Kulturmediums bzw. zum Verteilrohr stehen. Besonders günstig ist, wenn die Längsachsen der Steig- bzw. Fallrohre im Wesentlichen normal (bzw. normal) zum Spiegel des Kulturmediums bzw. zum (bevorzugt im Wesentlichen horizontalen) Verteilrohr stehen (mit anderen Worten: wenn diese vertikal orientiert sind). Zweckmäßigerweise sind die Längsachsen der Steig- und Fallrohre (insbesondere derer, die jeweils durch ein Verbindungsstück verbunden sind) zueinander im Wesentlichen parallel.

Das Verteilrohr ("Manifold") weist gemäß der erfindungsgemäßen Definition zumindest drei flüssigkeitsdurchlässige Anschlüsse für Steig- bzw. Fallrohre auf. Diese können z.B. als Bohrungen, Öffnungen oder Anschlussfortsätze (die genannte Grenzfläche muss diesfalls jedoch im Inneren des Verteilrohres selbst (mit anderen Worten: im Hauptrohr des Verteilrohres) verortet sein, um eine vereinfachte Reinigung sicherzustellen) ausgebildet sein. Es ist günstig, wenn das Verteilrohr entlang seiner Längsachse kein Gefälle bzw. keine Steigung mit einem Winkel (in Bezug auf den Spiegel des Kulturmediums) von mehr als 10°, bevorzugt von mehr als 5°, mehr bevorzugt von mehr als 2,5°, noch mehr bevorzugt von mehr als 1° oder gar von mehr als 0,5° aufweist. Besonders bevorzugt ist das Verteilrohr (entlang seiner Längsachse) im Wesentlichen horizontal oder horizontal orientiert (d.h. seine Längsachse ist im Wesentlichen parallel bzw. parallel zum Spiegel des Kulturmediums).

Im Kontext der Erfindung ist der Gasraum im Verteilrohr (der sich oberhalb des Kulturmediums im Verteilrohr befindet) bevorzugt ein Gasraum, der sich entlang der Längsachse des Verteilrohres über zumindest 10%, bevorzugt zumindest 20% oder gar zumindest 30%, mehr bevorzugt zumindest 40% oder gar zumindest 50%, noch mehr bevorzugt zumindest 60% oder gar zumindest 70%, insbesondere zumindest 80% oder gar zumindest 90% der Länge des Verteilrohres erstreckt. Besonders zweckmäßig ist es, wenn sich der genannte Gasraum über die gesamte Länge des Verteilrohres erstreckt. Es versteht sich, dass unter "Gas" auch ein Gasgemisch verstanden werden kann.

Um die Reinigung zu erleichtern, weisen das Verteilrohr und/oder die Steig- bzw. Fallrohre zweckmäßigerweise, gegebenenfalls unabhängig voneinander, entlang ihrer Längsachse keine Biegung von mehr als 90°, bevorzugt von mehr als 70°, mehr bevorzugt von mehr als 50°, noch mehr bevorzugt von mehr als 30° oder gar von mehr als 20°, insbesondere von mehr als 10° oder gar von mehr als 5° auf. Es ist besonders günstig, wenn das Verteilrohr und/oder die Steig- bzw. Fallrohre entlang ihrer Längsachse im Wesentlichen gerade oder gerade sind. Bevorzugt haben das Verteilrohr und/oder die Steig- bzw. Fallrohre (gegebenenfalls bis auf etwaige Anschlussfortsätze) ein im Wesentlichen rundes Profil.

Für den gesamten Kontext der Erfindung gilt, dass ein Verbindungsstück vorzugsweise genau eines der Steigrohre mit genau einem der Fallrohre flüssigkeitsdurchlässig verbindet (bzw. die beiden aneinander flüssigkeitsdurchlässig anschließt). Bevorzugt ist dabei, dass zumindest alle bis auf ein oder zwei Steigrohre des Reaktorelements und/oder zumindest alle bis auf ein oder zwei Fallrohre des Reaktorelements je paarweise mit einem solchen Verbindungsstück flüssigkeitsdurchlässig verbunden sind, insbesondere sind zumindest alle bis auf ein Steigrohr des Reaktorelements und/oder zumindest alle bis auf ein Fallrohr des Reaktorelements je paarweise mit einem solchen Verbindungsstück flüssigkeitsdurchlässig verbunden. Es ist konstruktiv günstig (insbesondere zur Unterstützung einer mäanderförmigen Strömung), wenn ein Verbindungsstück ein Steigrohr und ein Fallrohr, die zueinander benachbart sind (d.h. benachbart an das Verteilrohr angeschlossen sind), miteinander flüssigkeitsdurchlässig verbindet.

Unter "Betriebszustand" ist hierin ein Zustand des Photobioreaktors zu verstehen, in dem lebende phototrophe Mikroorganismen in Kulturmedium im Photobioreaktor kultiviert werden, wobei das Kulturmedium im Photobioreaktor vorzugsweise eine Strömung (insbesondere im Wesentlichen verursacht durch die Begasung) aufweist.

Die vorliegende Erfindung betrifft ferner die folgenden Aspekte bzw. Ausführungsformen davon:
Ausführungsform 1. Photobioreaktor zur Kultivierung phototropher Mikroorganismen, mit einem Reaktorelement, das ein Rohr aufweist, und mit einer Instandhaltungsvorrichtung und einem Antriebssystem, das die Instandhaltungsvorrichtung im Rohr bewegen kann, wobei der Photobioreaktor derart ausgelegt ist, dass das Rohr im Betriebszustand des Photobioreaktors von flüssigem Kulturmedium mit den Mikroorganismen zumindest teilweise durchströmt wird, dadurch gekennzeichnet, dass der Photobioreaktor derart ausgelegt ist, dass die Instandhaltungsvorrichtung im Betriebszustand des Photobioreaktors im Rohr einsetzbar ist und vom Antriebssystem zumindest gegen die Strömung des Kulturmediums im Rohr bewegt werden kann.

Ausführungsform 2. Photobioreaktor gemäß Ausführungsform 1, dadurch gekennzeichnet, dass der Photobioreaktor ferner eine Einrichtung zur Begasung des Kulturmediums aufweist, wobei der Photobioreaktor derart ausgelegt ist, dass im Betriebszustand des Photobioreaktors die Instandhaltungsvorrichtung im Rohr mit im Kulturmedium befindlichen Gasblasen in Berührung gebracht wird.

Ausführungsform 3. Photobioreaktor gemäß Ausführungsform 1 oder 2, dadurch gekennzeichnet, dass im Betriebszustand des Photobioreaktors die Instandhaltungsvorrichtung im Rohr für das Kulturmedium umströmbar und/oder durchströmbar ist.

Ausführungsform 4. Photobioreaktor gemäß einer der Ausführungsformen 1 bis 3, dadurch gekennzeichnet, dass der Photobioreaktor derart ausgelegt ist, dass im Betriebszustand im Rohr sowohl Kulturmedium als auch, oberhalb des Kulturmediums, ein Gasraum zur Aufnahme von aus dem Kulturmedium aufsteigenden Gasblasen vorhanden sind, wobei im Rohr eine Grenzfläche zwischen dem Kulturmedium und dem Gasraum angeordnet ist, und die Instandhaltungsvorrichtung zumindest zur Reinigung von mit dem Gasraum in Berührung befindlicher innerer Oberfläche des Rohres eingerichtet ist.

Ausführungsform 5. Photobioreaktor gemäß einer der Ausführungsformen 1 bis 4, dadurch gekennzeichnet, dass die Instandhaltungsvorrichtung eine Sprühdüse zum Besprühen der inneren Oberfläche des Rohres mit einer Reinigungsflüssigkeit aufweist, vorzugsweise wobei die Instandhaltungsvorrichtung zumindest zum Besprühen von mit dem genannten Gasraum in Berührung befindlicher innerer Oberfläche des Rohres durch die Sprühdüse eingerichtet ist.

Ausführungsform 6. Photobioreaktor gemäß einer der Ausführungsformen 1 bis 5, dadurch gekennzeichnet, dass die Instandhaltungsvorrichtung zumindest ein Wischblatt aus einem elastischen Material zum Wischen der inneren Oberfläche des Rohres aufweist, vorzugsweise wobei die Instandhaltungsvorrichtung zumindest zum Wischen von mit dem genannten Gasraum in Berührung befindlicher innerer Oberfläche des Rohres mit dem Wischblatt eingerichtet ist und/oder vorzugsweise wobei die Instandhaltungsvorrichtung eine, gegebenenfalls weitere, Sprühdüse aufweist, die zum Besprühen des Wischblatts mit einer Reinigungsflüssigkeit eingerichtet ist.

Ausführungsform 7. Photobioreaktor gemäß Ausführungsform 5 oder 6, wobei die Sprühdüse über eine Leitung mit einem Flüssigkeitsreservoir außerhalb des Rohres verbunden ist.

Ausführungsform 8. Photobioreaktor gemäß einer der Ausführungsformen 1 bis 8, dadurch gekennzeichnet, dass das Rohr ein Verteilrohr mit zumindest drei Anschlüssen für den Zufluss bzw. Abfluss von Kulturmedium ist, und der Photobioreaktor derart ausgelegt ist, dass die Instandhaltungsvorrichtung zu zumindest einem der Anschlüsse im Rohr bewegt werden kann und diesen abdichten kann.

Ausführungsform 9. Photobioreaktor gemäß Ausführungsform 8, dadurch gekennzeichnet, dass das Reaktorelement ferner eine Vielzahl von Steig- und Fallrohren für das flüssiges Kulturmedium aufweist, wobei die Steig- und Fallrohre jeweils an ihrem oberen Ende an das als Verteilrohr ausgebildete Rohr flüssigkeitsdurchlässig angeschlossen sind, wobei zumindest eines der Steig- und eines der Fallrohre zusätzlich miteinander durch ein Verbindungsstück flüssigkeitsdurchlässig verbunden sind, und wobei der Photobioreaktor derart ausgelegt ist, dass die Instandhaltungsvorrichtung gleichzeitig den Anschluss für das Fallrohr und den Anschluss für das Steigrohr, welche beide miteinander zusätzlich durch ein Verbindungsstück flüssigkeitsdurchlässig verbunden sind, abdichten kann.

Ausführungsform 10. Photobioreaktor gemäß Ausführungsform 9, dadurch gekennzeichnet, dass die Instandhaltungsvorrichtung dazu eingerichtet ist, das durch die Instandhaltungsvorrichtung gegenüber dem Rohr abgedichtete Fallrohr und das durch die Instandhaltungsvorrichtung gegenüber dem Rohr abgedichtete Steigrohr, welche beide miteinander durch ein Verbindungsstück flüssigkeitsdurchlässig verbunden sind, zu reinigen.

Ausführungsform 11. Photobioreaktor gemäß einer der Ausführungsformen 1 bis 10, dadurch gekennzeichnet, dass am Rohr, bevorzugt an dessen Außenseite, eine Führungsleiste zur Stabilisierung und/oder Positionierung der Instandhaltungsvorrichtung im Rohr vorgesehen ist.

Ausführungsform 12. Photobioreaktor gemäß einer der Ausführungsformen 1 bis 11, dadurch gekennzeichnet, dass das Antriebssystem zumindest eine Seilwinde, die ein im Rohr geführtes und mit der Instandhaltungsvorrichtung verbundenes Seil antreiben kann, aufweist, vorzugsweise wobei die Seilwinde mit einer Einrichtung zur Desinfektion des Seiles ausgestattet ist.

Ausführungsform 13. Photobioreaktor gemäß einer der Ausführungsformen 1 bis 12, dadurch gekennzeichnet, dass die Instandhaltungsvorrichtung zumindest zwei Module aufweist, die durch eine Kupplung miteinander verbunden sind, vorzugsweise wobei das Rohr im Wesentlichen aus Glas besteht.

Ausführungsform 14. Photobioreaktor gemäß einer der Ausführungsformen 1 bis 13, dadurch gekennzeichnet, dass der Photobioreaktor eine Station für die Instandhaltungsvorrichtung aufweist, die zur Reinigung der Instandhaltungsvorrichtung eingerichtet ist und in welche die Instandhaltungsvorrichtung bewegt werden kann.

Ausführungsform 15. Verfahren zur Kultivierung phototropher Mikroorganismen in einem Photobioreaktor, wobei der Photobioreaktor mit einem Reaktorelement, das ein Rohr aufweist, und mit einer Instandhaltungsvorrichtung im Rohr und einem Antriebssystem, das die Instandhaltungsvorrichtung im Rohr bewegt, ausgestattet ist, wobei das Rohr von flüssigem Kulturmedium mit den Mikroorganismen zumindest teilweise durchströmt wird, dadurch gekennzeichnet, dass das Verfahren den Einsatz der Instandhaltungsvorrichtung im Rohr im Betriebszustand des Photobioreaktors umfasst und die Instandhaltungsvorrichtung vom Antriebssystem zumindest gegen die Strömung des Kulturmediums im Rohr bewegt wird. vorzugsweise wobei der Photobioreaktor ferner gemäß einer der Ausführungsformen 1 bis 11 definiert ist.

Ausführungsform 16. Bewegliche Instandhaltungsvorrichtung für die Instandhaltung der inneren Oberfläche eines Rohres eines Photobioreaktors zur Kultivierung phototropher Mikroorganismen, dadurch gekennzeichnet, dass die Instandhaltungsvorrichtung für die Instandhaltung der inneren Oberfläche des Rohres im laufenden Betrieb des Photobioreaktors, während das Rohr von flüssigem Kulturmedium mit den Mikroorganismen zumindest teilweise durchströmt wird, geeignet ist und dafür eingerichtet ist, von einem externen Antriebssystem, welches bevorzugt eine Seilwinde aufweist, zumindest gegen die Strömung des Kulturmediums im Rohr bewegt zu werden.

Ausführungsform 17. Bewegliche Instandhaltungsvorrichtung gemäß Ausführungsform 16, dadurch gekennzeichnet, dass die Instandhaltungsvorrichtung eine Sprühdüse zum Besprühen der inneren Oberfläche des Rohres mit einer Reinigungsflüssigkeit und einen Anschluss für eine Leitung mit der Flüssigkeit aufweist.

Ausführungsform 18. Bewegliche Instandhaltungsvorrichtung gemäß Ausführungsform 16 oder 17, dadurch gekennzeichnet, dass die Instandhaltungsvorrichtung zumindest ein Wischblatt aus einem elastischen Material zum Wischen der inneren Oberfläche des Rohres aufweist, vorzugsweise wobei die Instandhaltungsvorrichtung eine, gegebenenfalls weitere, Sprühdüse aufweist, die zum Besprühen des Wischblatts mit einer Reinigungsflüssigkeit eingerichtet ist.

Ausführungsform 19. Bewegliche Instandhaltungsvorrichtung gemäß einer der Ausführungsformen 16 bis 18, dadurch gekennzeichnet, dass die Instandhaltungsvorrichtung zumindest zwei Module aufweist, die durch eine Kupplung miteinander verbunden sind.

Ausführungsform 20. Bewegliche Instandhaltungsvorrichtung gemäß einer der Ausführungsformen 16 bis 19, dadurch gekennzeichnet, dass die Instandhaltungsvorrichtung ferner mit einer Einrichtung zum Abdichten von Anschlüssen des Rohres ausgestattet ist.

Ausführungsform 21. Verwendung der beweglichen Instandhaltungsvorrichtung gemäß einer der Ausführungsformen 16 bis 20 zur Instandhaltung der inneren Oberfläche eines Rohres eines Photobioreaktors zur Kultivierung phototropher Mikroorganismen, wobei die Instandhaltung im laufenden Betrieb des Photobioreaktors, wobei das Rohr von flüssigem Kulturmedium mit den Mikroorganismen zumindest teilweise durchströmt wird, erfolgt.

Die Erfindung wird nachfolgend anhand von besonders bevorzugten Ausführungsbeispielen, auf die sie jedoch nicht eingeschränkt ist, und unter Bezugnahme auf Zeichnungen noch weiter erläutert. Die Zeichnungen zeigen im Einzelnen:
**Fig. 1** zeigt einen Photobioreaktor gemäß Stand der Technik (US 2011/0027875 A1), bei dem die Grenzflächen zwischen Kulturmedium und Gasraum unterhalb des oberen Gasverteilrohres verortet sind;
**Fig. 2** zeigt eine Draufsicht auf eine Ausführung des erfindungsgemäßen Photobioreaktors im Betriebszustand;
**Fig. 3** zeigt eine Draufsicht auf eine Ausführung des erfindungsgemäßen Photobioreaktors mit Instandhaltungsvorrichtung im Betriebszustand;
**Fig. 4** ist eine lokal vergrößerte Ansicht der Ausführungen gemäß Fig. 2 bzw. Fig. 3;
**Fig. 5A** zeigt eine Draufsicht auf ein befülltes Verteilrohr, wie es für die vorliegenden Erfindung verwendet werden kann;
**Fig. 5B** zeigt eine perspektivische Ansicht des Verteilrohres gemäß Fig. 5A;
**Fig. 6** zeigt eine perspektivische Ansicht eines Metallgerüsts, wie es für Ausführungen von Reaktorelementen des erfindungsgemäßen Photobioreaktors werden kann;
**Fig. 7** zeigt eine perspektivische Ansicht von Reaktorelementen einer Ausführungsform des erfindungsgemäßen Photobioreaktors.

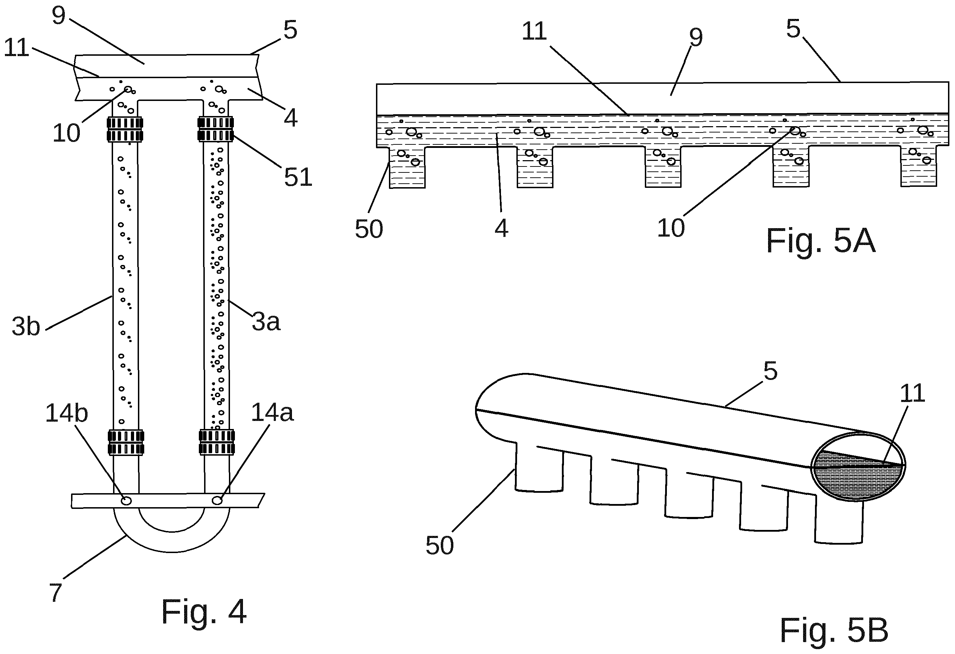

Fig. 2 zeigt einen Photobioreaktor 1 zur Kultivierung von phototrophen Mikroorganismen, mit zwei Reaktorelementen 2, die jeweils eine Vielzahl von vertikalen, geraden Steigrohren 3a und Fallrohren 3b für Kulturmedium 4 und ein horizontales, gerades Verteilrohr 5 aufweisen. Das Kulturmedium 4 ist ein flüssiges Kulturmedium auf Wasserbasis und angereichert mit Nährstoffen; es beinhaltet die phototrophen Mikroorganismen (üblicherweise Mikroalgen). Die genannten Rohre bestehen aus Glas, wobei die Verteilrohre 5 mit Anschlussfortsätzen 50 vorgesehen sind (vgl. auch Fig. 5A und Fig. 5B). Die Steigrohre 3a und Fallrohre 3b sind jeweils an ihrem oberen Ende 6 mit Hilfe der Rohrverbinder 51 mit den Anschlussfortsätzen 50 des Verteilrohres 5 flüssigkeitsdurchlässig verbunden. Die Steigrohre 3a und die Fallrohre 3b sind zusätzlich miteinander durch je ein U-förmiges Verbindungsstück 7 flüssigkeitsdurchlässig verbunden (vgl. auch Fig. 4).

Der Photobioreaktor 1 weist ferner eine Einrichtung 8 zum Einleiten von Kohlendioxid (und/oder Druckluft) in die Steigrohre 3a bzw. die Fallrohre 3b auf. Für das mit dem Fallrohr 3b durch das Verbindungsstück 7 verbundene Steigrohr 3a ist ein Einlass 14a zur Begasung durch die Einrichtung 8 vorgesehen. Für das mit dem Steigrohr 3a durch das Verbindungsstück 7 verbundene Fallrohr 3b ist ein weiterer Einlass 14b zur Begasung durch die Einrichtung 8 vorgesehen. Das Kohlendioxid wird durch eine Gaspumpe aus einem Flüssigtank in ein Gasverteilrohr gepumpt. Die Anschlüsse des Gasverteilrohres sind durch Schläuche mit je einem Einlass 14a bzw. 14b verbunden. Durch Vorsehen einer Öffnung des Einlasses 14a, die durchlässiger ist als die Öffnung des Einlasses 14b, wird eine stärkere Begasung der Steigrohre 3a gegenüber den Fallrohren 3b ermöglicht. Dadurch kommt es - überraschenderweise obwohl am jeweiligen oberen Ende 6 der Steigrohre 3a bzw. Fallrohre 3b keine U-förmigen Verbindungsstücke 7 sondern das Verteilrohr 5 vorgesehen ist - zu einer mäanderförmigen Strömung des Kulturmediums 4 (aufwärts in den Steigrohren 3a, abwärts in den Fallrohren 3b; vgl. auch die gestrichelten Pfeile in Fig. 3). Diese mäanderförmige Strömung wird darüber hinaus weiter stabilisiert, indem die zwei Reaktorelemente 2 über ein Fallrohr 3b des ersten Reaktorelements 2 mit einem Steigrohr 3a des zweiten Reaktorelements 2 miteinander durch ein U-förmiges Verbindungsstück 7 flüssigkeitsdurchlässig verbunden sind - also vereinfacht gesagt durch Vorsehen einer "Lücke" zwischen den beiden Verteilrohren 5.

Der Einlass 52a und der Auslass 52b können durch einen Schlauch miteinander verbunden werden, wodurch ein zyklischer Betrieb des Photobioreaktors 1 ermöglicht wird. Nach einer gewissen Anzahl an Zyklen unter Belichtung kann das Kulturmedium 4, das nun eine deutlich höhere Konzentration an Mikroorganismen enthält, am Auslass 52b zur Ernte (d.h. zur Aufkonzentration und Trocknung der phototrophen Mikroorganismen) entnommen werden, während am Einlass 52a frisches Kulturmedium 4 mit einer niedrigen Anfangskonzentration an phototrophen Mikroorganismen eingebracht wird. Selbstverständlich ist auch ein kontinuierlicher zyklischer Betrieb denkbar, bei dem der Einlass 52a mit dem Auslass 52b durch einen Schlauch verbunden bleibt, und an einem Einlass in einem ersten unteren Verbindungsstück 7 kontinuierlich frisches Kulturmedium 5 zugeführt wird und an einem Auslass in einem ausgehend von der Strömungsrichtung direkt davor liegenden zweiten unteren Verbindungsstück 7 kontinuierlich dieselbe Menge dichteres Kulturmedium 4 entnommen wird. Dafür ist naturgemäß eine gewisse Mindestlänge der Zuchtstrecke erforderlich. Ebenso ist, wenn der Photobioreaktor 1 eine gewisse Mindestlänge aufweist, auch ein kontinuierlicher linearer Betrieb denkbar, bei dem kontinuierlich frisches Kulturmedium 4 mit einer niedrigen Anfangskonzentration an phototrophen Mikroorganismen beim Einlass 52a eingebracht wird und kontinuierlich dieselbe Menge reifes Kulturmedium 4 am Auslass 52b zur Ernte entnommen wird. Einlässe können jedoch auch an anderen Stellen des Reaktors vorgesehen werden (z.B. U-Bogen-Verbindungsstück des ersten/letzten Steig- bzw. Fallrohres, aber auch in einem anderen U-Bogen-Verbindungsstück im Photobioreaktor).

Das mäanderförmig strömende Kulturmedium 4 mit den phototrophen Mikroorganismen steht in den Reaktorelementen 2 so hoch, dass auch die Verteilrohre 5 jeweils etwa zur Hälfte damit befüllt sind. Im Verteilrohr 5 sind also sowohl Kulturmedium 4 als auch, oberhalb des Kulturmediums 4, ein Gasraum 9 zur Aufnahme von aus dem Kulturmedium 4 aufsteigenden Gasblasen 10 vorhanden, wobei im Verteilrohr 5 eine Grenzfläche 11 zwischen dem Kulturmedium 4 und dem Gasraum 9 angeordnet ist. Der Gasdruckausgleich mit der Umgebung findet über das Ventil 53 statt, welches mit einem Filtersystem ausgestattet ist, um Kontamination des Kulturmediums 4 zu vermeiden.

Die mit der Grenzfläche 11 zwischen Kulturmedium 4 und Gasraum 9 in Zusammenhang stehenden Verschmutzungen an der Innenoberfläche des Verteilrohres 5 können mit verhältnismäßig geringem Aufwand gereinigt werden, indem der Photobioreaktor 1 kurz außer Betrieb genommen wird, das Kulturmedium 4 aus dem Verteilrohr 5 abgelassen wird, die Abdeckplatte 54 des Verteilrohres entfernt wird und die Innenoberfläche das Verteilrohres z.B. mit einer an einer Teleskopstange befestigten Bürste gereinigt wird.

Die in Fig. 3 gezeigte Ausführung des Photobioreaktors 1 ermöglicht die Reinigung bzw. das Sauberhalten der inneren Oberfläche des Verteilrohres 5 während des Betriebes. Dabei ist am Verteilrohr 5 die Führungsschiene 12 für die bewegliche Instandhaltungsvorrichtung 13 vorgesehen. Die Instandhaltungsvorrichtung 13 ist mit Bürsten und Sprühdüsen für die inneren Oberfläche des Verteilrohres 5 ausgestattet. Die Instandhaltungsvorrichtung 13 ist z.B. magnetisch und kann durch einen in der Führungsschiene 12 verschiebbaren Magneten (oder Seile) bewegt werden. Die Instandhaltungsvorrichtung 13 ist umströmbar und kann während des laufenden Betriebes in voreinstellbaren Zeitabständen eingesetzt werden, was die Stehzeiten des Photobioreaktors 1 wesentlich verringert. Die gestrichelten Pfeile verdeutlichen den Strömungspfad im Reaktorelement 2.

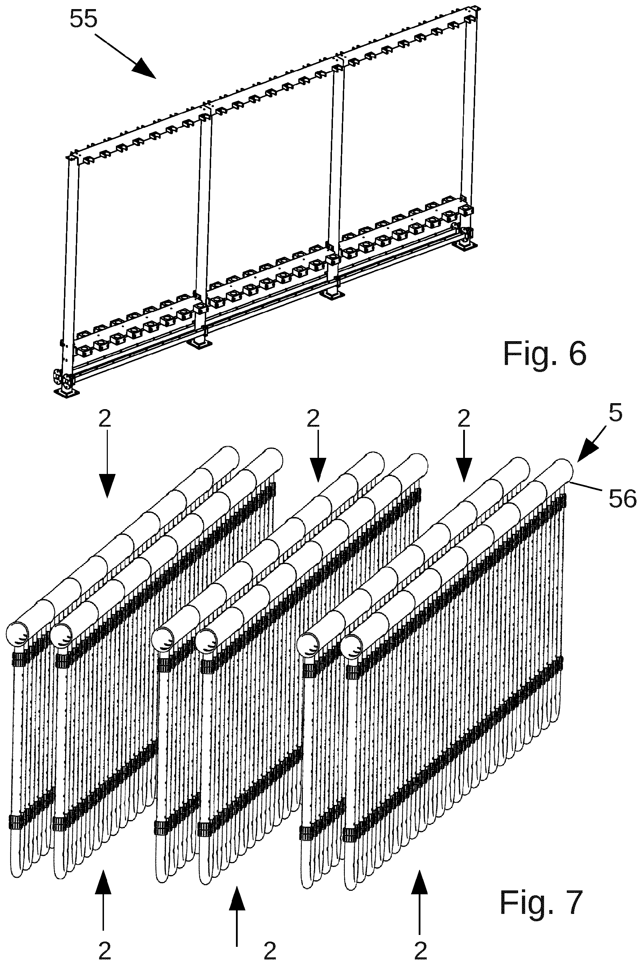

Fig. 7 zeigt schematisch eine Ausführungsform des Photobioreaktors 1, bei der das jeweilige Verteilrohr 5 eines Reaktorelements 2 aus miteinander direkt verbundenen Glasteilstücken 56 besteht. Jeweils zwei Reaktorelemente 2 werden durch das in Fig. 6 gezeigte Metallgerüst 55 gehalten.

## Patentansprüche

1. Photobioreaktor (1) zur Kultivierung phototropher Mikroorganismen,
mit einem Reaktorelement (2), das eine Vielzahl von Steig- (3a) und Fallrohren (3b) für flüssiges Kulturmedium (4), welches die Mikroorganismen beinhaltet, und ein Verteilrohr (5) aufweist, wobei die Steig- (3a) und Fallrohre (3b) jeweils an ihrem oberen Ende (6) mit dem Verteilrohr (5) flüssigkeitsdurchlässig verbunden sind, wobei zumindest eines der Steig- (3a) und eines der Fallrohre (3b) zusätzlich miteinander durch ein Verbindungsstück (7) flüssigkeitsdurchlässig verbunden sind,
wobei der Photobioreaktor (1) ferner eine Einrichtung (8) zum Einleiten von Gas in zumindest ein Steigrohr (3a) aufweist,
**dadurch gekennzeichnet, dass** der Photobioreaktor (1) derart ausgelegt ist, dass im Betriebszustand im Verteilrohr (5) sowohl Kulturmedium (4) als auch, oberhalb des Kulturmediums (4), ein Gasraum (9) zur Aufnahme von aus dem Kulturmedium (4) aufsteigenden Gasblasen (10) vorhanden sind, wobei im Verteilrohr (5) eine Grenzfläche (11) zwischen dem Kulturmedium (4) und dem Gasraum (9) angeordnet ist.

2. Photobioreaktor (1) gemäß Anspruch 1, **dadurch gekennzeichnet, dass** zumindest zwei Reaktorelemente (2) vorgesehen sind, wobei ein Fallrohr (3b) des ersten Reaktorelements (2) mit einem Steigrohr (3a) des zweiten Reaktorelements (2) miteinander durch ein Verbindungsstück (7) flüssigkeitsdurchlässig verbunden sind.

3. Photobioreaktor (1) gemäß Anspruch 1 oder 2, **dadurch gekennzeichnet, dass** die Steig- (3a) und Fallrohre (3b) und/oder das Verteilrohr (5) im Wesentlichen aus Glas bestehen.

4. Photobioreaktor (1) gemäß einem der Ansprüche 1 bis 3, **dadurch gekennzeichnet, dass** am Verteilrohr (5) eine Einrichtung (12) zur Führung einer im Verteilrohr (5) beweglichen Instandhaltungsvorrichtung (13) vorgesehen ist.

5. Photobioreaktor (1) gemäß einem der Ansprüche 1 bis 4, **dadurch gekennzeichnet, dass** für das mit dem Fallrohr (3b) durch das Verbindungsstück (7) verbundene Steigrohr (3a) ein Einlass (14a) zur Begasung mit der Einrichtung (8) vorgesehen ist, und dass für das mit dem Steigrohr (3a) durch das Verbindungsstück (7) verbundene Fallrohr (3b) ein weiterer Einlass (14b) zur Begasung mit der Einrichtung (8) vorgesehen ist.

6. Photobioreaktor (1) gemäß Anspruch 5, **dadurch gekennzeichnet, dass** zumindest einer der Einlässe zur Begasung (14a,14b) im Verbindungsstück (7) vorgesehen ist.

7. Photobioreaktor (1) gemäß Anspruch 5 oder 6, **dadurch gekennzeichnet, dass** er derart ausgelegt ist, dass im Betriebszustand eine Agitation und/oder ein Fluss des Kulturmediums (4) im Wesentlichen durch die im Kulturmedium (4) aufsteigenden Gasblasen (10) verursacht wird.

8. Photobioreaktor (1) gemäß Anspruch 7, **dadurch gekennzeichnet, dass** er derart ausgelegt ist, dass im Betriebszustand eine mäanderförmiger Strömung des Kulturmediums (4) durch die Steig-(3a) und Fallrohre (3b) dadurch erreicht wird, dass das Kulturmedium (4) im mit dem Fallrohr (3b) durch das Verbindungsstück (7) verbundenen Steigrohr (3a) stärker begast wird als in dem Fallrohr (3b).

9. Verfahren zur Kultivierung phototropher Mikroorganismen, umfassend das Belichten eines Photobioreaktors (1),
wobei der Photobioreaktor (1) versehen ist mit einem Reaktorelement (2), das eine Vielzahl von Steig- (3a) und Fallrohren (3b) für flüssiges Kulturmedium (4), welches die Mikroorganismen beinhaltet, und ein Verteilrohr (5) aufweist, wobei die Steig-(3a) und Fallrohre (3b) jeweils an ihrem oberen Ende (6) mit dem Verteilrohr (5) flüssigkeitsdurchlässig verbunden sind, wobei zumindest eines der Steig- (3a) und eines der Fallrohre (3b) zusätzlich miteinander durch ein Verbindungsstück (7) flüssigkeitsdurchlässig verbunden sind,
und wobei der Photobioreaktor (1) in den Steig- (3a) und Fallrohren (3b) flüssiges Kulturmedium (4) mit den Mikroorganismen beinhaltet, welches zumindest teilweise begast wird,
**dadurch gekennzeichnet, dass** im Verteilrohr (5) sowohl flüssiges Kulturmedium (4) mit den Mikroorganismen als auch, oberhalb des Kulturmediums (4), ein Gasraum (9) vorhanden sind, wobei im Verteilrohr (5) eine Grenzfläche (11) zwischen dem Kulturmedium (4) und dem Gasraum (9) ausgebildet wird, und der Gasraum (9) aus dem Kulturmedium (4) aufsteigende Gasblasen (10) aufnimmt.

10. Verfahren gemäß Anspruch 9, **dadurch gekennzeichnet, dass** der Photobioreaktor der Photobioreaktor gemäß einem der Ansprüche 1 bis 8 ist.

11. Verfahren gemäß Anspruch 9 oder 10, **dadurch gekennzeichnet, dass** das Kulturmedium (4) mäanderförmig durch die Steig- (3a) und Fallrohre (3b) strömt.

12. Verfahren gemäß einem der Ansprüche 8 bis 11, **dadurch gekennzeichnet, dass** die innere Oberfläche des Verteilrohres (5) von der beweglichen Instandhaltungsvorrichtung (13), vorzugsweise in voreinstellbaren Zeitabständen, gereinigt wird.

## Claims

1. A photobioreactor (1) for the cultivation of phototrophic microorganisms,
having a reactor element (2) that comprises a plurality of risers (3a) and down pipes (3b) for liquid culture medium (4) that contains the microorganisms, and a manifold (5), wherein the risers (3a) and down pipes (3b) are each connected in a liquid-permeable manner at their upper end (6) to the manifold (5), wherein at least one of the risers (3a) and one of the down pipes (3b) are additionally connected to each other in a liquid-permeable manner by a connecting piece (7),
wherein the photobioreactor (1) further comprises a device (8) for introducing gas into at least one riser (3a),
**characterized in that** the photobioreactor (1) is designed such that in the operating state in the manifold (5), both culture medium (4) and, above the culture medium (4), a gas space (9) for receiving gas bubbles (10) rising from the cultural medium (4), are present, wherein an interface (11) is arranged between the culture medium (4) and the gas space (9) in the manifold (5).

2. The photobioreactor (1) according to claim 1, **characterized in that** at least two reactor elements (2) are provided, wherein a down pipe (3b) of the first reactor element (2) and a riser (3a) of the second reactor element (2) are connected to each other in a liquid-permeable manner by a connecting piece (7).

3. The photobioreactor (1) according to claim 1 or 2, **characterized in that** the risers (3a) and down pipes (3b) and/or the manifold (5) are made substantially of glass.

4. The photobioreactor (1) according to one of claims 1 to 3, **characterized in that** a device (12) for guiding a maintenance device (13) which is movable in the manifold (5) is provided on the manifold (5).

5. The photobioreactor (1) according to one of claims 1 to 4, **characterized in that** an inlet (14a) for gassing with the device (8) is provided for the riser (3a) connected to the down pipe (3b) through the connecting piece (7), and that a further inlet (14b) is provided for gassing with the device (8) for the down pipe (3b) connected to the riser (3a) by the connecting piece (7).

6. The photobioreactor (1) according to claim 5, **characterized in that** at least one of the inlets for gassing (14a, 14b) is provided in the connecting piece (7).

7. The photobioreactor (1) according to claim 5 or 6, **characterized in that** it is designed such that in the operating state, agitation and/or flow of the culture medium (4) is substantially caused by the gas bubbles (10) rising in the culture medium (4).

8. The photobioreactor (1) according to claim 7, **characterized in that** it is designed such that in the operating state, a meandering flow of the culture medium (4) through the risers (3a) and down pipes (3b) is achieved, **in that** the culture medium (4) in the riser (3a) connected to the down pipe (3b) through the connecting piece (7) is gassed stronger than in the down pipe (3b).

9. A method for cultivating phototrophic microorganisms, comprising exposing a photobioreactor (1) to light,
wherein the photobioreactor (1) is provided with a reactor element (2) which comprises a plurality of risers (3a) and down pipes (3b) for liquid culture medium (4) which contains the microorganisms, and a manifold (5), wherein the risers (3a) and down pipes (3b) each are connected in a liquid-permeable manner at their upper end (6) to the manifold (5), wherein at least one of the risers (3a) and one of the down pipes (3b) are additionally connected to each other in a liquid-permeable manner by a connecting piece (7),
and wherein the photobioreactor (1) in the risers (3a) and down pipes (3b) contains liquid culture medium (4) having the microorganisms, which culture medium is at least partially gassed,
**characterized in that** in the manifold (5), both liquid culture medium (4) having the microorganisms and, above the culture medium (4), a gas space (9) are present, wherein in the manifold (5), an interface (11) is formed between the culture medium (4) and the gas space (9), and the gas space (9) receives gas bubbles (10) rising from the culture medium (4).

10. The method according to claim 9, **characterized in that** the photobioreactor is the photobioreactor according to one of claims 1 to 8.

11. The method according to claim 9 or 10, **characterized in that** the culture medium (4) flows meandering through the risers (3a) and down pipes (3b).

12. The method according to one of claims 8 to 11, **characterized in that** the inner surface of the manifold (5) is cleaned by the mobile maintenance device (13), preferably at pre-settable time intervals.

## Revendications

1. Photobioréacteur (1) pour la culture de micro-organismes phototrophes, comprenant un élément de réacteur (2) qui présente une pluralité de tubes ascendants (3a) et descendants (3b) pour un milieu de culture liquide (4), qui contient des micro-organismes, et un tube répartiteur (5), les tubes ascendants (3a) et descendants (3b) étant raccordés respectivement à leur extrémité supérieure (6) au tube répartiteur (5) de manière perméable aux liquides, au moins l'un des tubes ascendants (3a) et l'un des tubes descendants (3b) étant raccordés en outre l'un à l'autre par le biais d'une pièce de raccordement (7) de manière perméable aux liquides,
le photobioréacteur (1) présentant en outre un système (8) pour introduire des gaz dans au moins un tube ascendant (3a),
le photobioréacteur (1) étant **caractérisé en ce qu'**il est conçu de sorte que, à l'état en fonctionnement, il y ait du milieu de culture (4) dans le tube répartiteur (5) ainsi qu'une chambre de gaz (9), au-dessus du milieu de culture (4), destinée à la réception des bulles de gaz (10) montant depuis le milieu de culture (4), une interface (11) entre le milieu de culture (4) et la chambre de gaz (9) étant disposée dans le tube répartiteur (5).

2. Photobioréacteur (1) selon la revendication 1, **caractérisé en ce qu'**au moins deux éléments de réacteur (2) sont prévus, un tube descendant (3b) du premier élément de réacteur (2) étant relié réciproquement avec un tube ascendant (3a) du deuxième élément de réacteur (2) par le biais d'une pièce de raccordement (7) de manière perméable aux liquides.

3. Photobioréacteur (1) selon la revendication 1 ou 2, **caractérisé en ce que** les tubes ascendants (3a) et les tubes descendants (3b) et/ou le tube répartiteur (5) se composent sensiblement de verre.

4. Photobioréacteur (1) selon l'une des revendications 1 à 3, **caractérisé en ce qu'**un appareil (12) pour le guidage d'un dispositif d'entretien mobile (13) dans le tube répartiteur (5) est prévu sur le tube répartiteur (5).

5. Photobioréacteur (1) selon l'une des revendications 1 à 4, **caractérisé en ce qu'**un orifice d'entrée (14a) pour le gazage avec l'appareil (8) est prévu pour le tube ascendant (3a) relié avec le tube descendant (3b) par le biais de la pièce de raccordement (7), et qu'un autre orifice d'entrée (14b) pour le gazage avec l'appareil (8) est prévu pour le tube descendant (3b) relié avec le tube ascendant (3a) par le biais de la pièce de raccordement (7).

6. Photobioréacteur (1) selon la revendication 5, **caractérisé en ce qu'**au moins l'un des orifices d'entrée pour le gazage (14a, 14b) est disposé dans la pièce de raccordement (7).

7. Photobioréacteur (1) selon la revendication 5 ou 6, **caractérisé en ce qu'**il est conçu de sorte que, à l'état de fonctionnement, une agitation et/ou un flux de milieu de culture (4) soit sensiblement provoqué par les bulles de gaz (10) remontant dans le milieu de culture (4).

8. Photobioréacteur (1) selon la revendication 7, **caractérisé en ce qu'**il est conçu de sorte que, à l'état de fonctionnement, une circulation du milieu de culture (4) en forme de méandres à travers les tubes ascendants (3a) et descendants (3b) est obtenue en gazant plus fortement le milieu de culture (4) dans le tube ascendant (3a) relié au tube descendant (3b) par le biais de la pièce de raccordement (7) que dans le tube descendant (3b).

9. Procédé de culture de micro-organismes phototrophes, comprenant l'exposition à la lumière d'un photobioréacteur (1),
dans lequel le photobioréacteur (1) est doté d'un élément de réacteur (2) qui présente une pluralité de tubes ascendants (3a) et descendants (3b) pour un milieu de culture liquide (4), qui contient des micro-organismes, et un tube répartiteur (5), les tubes ascendants (3a) et descendants (3b) étant raccordés respectivement à leur extrémité supérieure (6) au tube répartiteur (5) de manière perméable aux liquides, au moins l'un des tubes ascendants (3a) et l'un des tubes descendants (3b) étant raccordés en outre l'un à l'autre par le biais d'une pièce de raccordement (7) de manière perméable aux liquides,
et dans lequel le photobioréacteur (1) contient dans les tubes ascendants (3a) et descendants (3b) un milieu de culture liquide (4) avec les micro-organismes, lequel est au moins partiellement gazé,
**caractérisé en ce qu'**il y a dans le tube répartiteur (5) aussi bien un milieu de culture liquide (4) avec les micro-organismes que, au-dessus du milieu de culture (4), une chambre de gaz (9), une interface (11) étant formée entre le milieu de culture (4) et la chambre de gaz (9) dans le tube répartiteur (5), et la chambre de gaz (9) recevant les bulles de gaz (10) remontant depuis le milieu de culture (4).

10. Procédé selon la revendication 9, **caractérisé en ce que** le photobioréacteur est le photobioréacteur selon l'une des revendications 1 à 8.

11. Procédé selon la revendication 9 ou 10, caractérisé en que le milieu de culture (4) circule en forme de méandres à travers les tubes ascendants (3a) et les tubes descendants (3b).

12. Procédé selon l'une des revendications 8 à 11, **caractérisé en ce que** la surface interne du tube répartiteur (5) est nettoyée par le dispositif d'entretien mobile (13), de préférence à des intervalles de temps pré-réglables.
